(19) Europäisches Patentamt
European Patent Office
Office européen des brevets

(11) Numéro de publication : **0 429 331 B1**

(12) # FASCICULE DE BREVET EUROPEEN

(45) Date de publication du fascicule du brevet : **12.05.93 Bulletin 93/19**

(51) Int. Cl.$^5$ : **C07C 19/08,** C07C 17/22

(21) Numéro de dépôt : **90403118.4**

(22) Date de dépôt : **05.11.90**

(54) **Synthèse des bromures de perfluoroalkyle.**

(30) Priorité : **24.11.89 FR 8915522**

(43) Date de publication de la demande : **29.05.91 Bulletin 91/22**

(45) Mention de la délivrance du brevet : **12.05.93 Bulletin 93/19**

(84) Etats contractants désignés : **AT BE CH DE DK ES FR GB GR IT LI LU NL SE**

(56) Documents cités :
**EP-A- 0 298 870**
**US-A- 3 456 024**

(73) Titulaire : **ELF ATOCHEM S.A.**
**4 & 8, Cours Michelet La Défense 10**
**F-92800 Puteaux (FR)**

(72) Inventeur : **Drivon, Gilles**
**3 Rue Joanny Courbière**
**F-69850 Saint-Martin-en-Haut (FR)**
Inventeur : **Durual, Pierre**
**Les Essarts No 2, Chemin du Rossignol**
**F-69390 Vernaison (FR)**
Inventeur : **Ghenassia, Elie**
**20 Avenue Albert 1er de Belgique**
**F-38100 Grenoble (FR)**

(74) Mandataire : **Leboulenger, Jean et al**
**ATOCHEM Département Propriété Industrielle**
**F-92091 Paris la Défense 10 Cédex 42 (FR)**

**EP 0 429 331 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne le domaine des hydrocarbures aliphatiques perhalogénés et a plus particulièrement pour objet la préparation des bromures de perfluoroalkyle ou bromoperfluoroalcanes $R_F$-Br, $R_F$ désignant un radical perfluoroalkyle $C_nF_{2n+1}$, linéaire ou ramifié, contenant de 1 à 20 atomes de carbone.

Ces composés connus sont utilisés dans de nombreux domaines, en particulier en médecine comme radiopaques (agents de contraste aux rayons X) ou comme transporteurs d'oxygène dans les substituts du sang. Un composé plus spécialement étudié dans ce domaine est le bromure de n.perfluorooctyle $C_8F_{17}Br$.

Parmi les méthodes connues pour préparer ces composés, on peut signaler plus particulièrement :

- l'action du brome sur un composé $R_F$-$SF_5$ à 500°C en présence de nickel (brevet US 3 456 024) ;
- la photolyse en phase gazeuse d'un mélange d'hydrogéno-1 perfluoroalcane et de BrCl ou BrF (Adcock et al., Chem. Abstr. 100 :34092e);
- l'action du brome sur les composés $R_F$-I en présence d'un initiateur radicalaire comme l'AIBN (demande japonaise Kokai 85-184033) ;
- la photobromation de ces mêmes composés iodés par irradiation aux UV (Huang et al., Huaxue Xuebao 1984, 42(10) 1106-8 résumé dans Chem. Abstr. 102 : 78312x).

La faiblesse des rendements obtenus et/ou la cinétique lente de ces méthodes font qu'elles ne permettent pas la production économique des bromures de perfluoroalkyle à l'échelle industrielle. Vu l'importance de ces composés dans le domaine médical, il est du plus grand intérêt de pouvoir les fabriquer au plus bas coût possible.

Dans la demande de brevet EP 0298870 on a décrit un procédé de fabrication des bromures de perfluoroalkyle à partir des chlorures de perfluoroalcane-sulfonyle correspondants $R_F$-$SO_2Cl$. Ce procédé qui correspond au schéma réactionnel suivant :

$$R_F\text{-}SO_2Cl + HBr \xrightarrow{cata/\Delta} R_FBr + SO_2 + HCl$$

consiste à faire réagir le bromure d'hydrogène gazeux sur un chlorure de perfluoroalcane-sulfonyle en présence d'un catalyseur constitué par une amine ou une phosphine tertiaire ou un sel d'ammonium ou de phosphonium quaternaire, à une température pouvant aller de 80 à 200°C (de préférence comprise entre 90 et 150°C) ; la quantité de catalyseur peut aller de 0,1 à 5 moles pour 100 moles de chlorure $R_F$-$SO_2Cl$ et est de préférence comprise entre environ 1 et 2 moles pour 100. Bien que ce procédé permette de fabriquer les bromures de perfluoroalkyle en une seule étape, avec un excellent rendement et une très bonne sélectivité, il nécessite l'emploi de HBr gazeux anhydre, un produit cher pour le fabricant qui ne dispose pas d'une installation spécifique de production de HBr sur son site industriel. D'autre part, dans certains cas, le caractère réducteur de HBr peut conduire à la formation concomitante d'impuretés soufrées (par exemple $R_F$-S-S-$R_F$ et $R_F$-SBr) et donc à une baisse de rendement.

Il a maintenant été trouvé que le bromure de tétrabutylammonium et plus généralement les composés de formule générale :

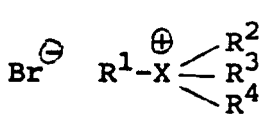

(I)

dans laquelle X représente un atome d'azote ou de phosphore, les symboles $R^1$, $R^2$, $R^3$ et $R^4$, identiques ou différents, représentent chacun un radical hydrocarboné éventuellement substitué, l'un de ces symboles pouvant aussi être un atome d'hydrogène et/ou trois d'entre eux ensemble avec X pouvant former un noyau pyridine, ont la propriété d'échanger très rapidement leur atome de brome avec l'atome de chlore d'un sulfochlorure $R_FSO_2Cl$ et de favoriser, même à basse température, la décomposition en $R_FBr$ du sulfobromure $R_FSO_2Br$ intermédiairement formé.

Ainsi, à condition d'utiliser une quantité au moins équimolaire de composé (I), il est possible de produire les bromures de perfluoroalkyle $R_F$F-Br à partir des sulochlorures $R_F$-$SO_2Cl$ sans avoir recours à l'emploi de HBr gazeux.

L'invention a donc pour objet un procédé de préparation des bromures de perfluoroalkyle, caractérisé en

ce qu'il consiste à faire réagir un chlorure de perfluoroalcane-sulfonyle avec une quantité au moins équimolaire d'un composé de formule générale (I).

Dans cette formule, les radicaux hydrocarbonés $R^1$ à $R^4$ peuvent être notamment des radicaux alkyle contenant de 1 à 16 atomes de carbone et, de préférence, 1 à 8 (par exemple, méthyl, éthyl, propyl, butyl, octyl), des radicaux aryle, de préférence phényle, ou des radicaux aralkyle, de préférence benzyle. Ces radicaux qui peuvent être identiques ou différents, peuvent porter un ou plusieurs substituants, pourvu qu'ils ne soient pas susceptibles de réagir avec le sulfochlorure de départ.

Un composé de formule (I) particulièrement préféré est le bromure de tétrabutylammonium (ci-après désigné par l'abréviation TBAB). Comme autres exemples de composés de formule (I) on peut citer, à titre non limitatif, le bromure de tétraméthylammonium, le bromure de tétrabutylphosphonium, le bromure de phényl triméthyl ammonium, le bromure de benzyl triméthyl ammonium, le bromhydrate de tripropylamine et le bromhydrate de pyridine.

Comme indiqué précédemment, le composé de formule (I) doit être utilisé en une quantité au moins équimolaire par rapport au sulfochlorure de départ. Pour accélérer la réaction, il est généralement avantageux d'utiliser le compose de formule (I) en léger excès (jusqu'à 10 % environ). On ne sortirait cependant pas du cadre de la présente invention en utilisant un excès de composé (I) supérieur à 10 %.

La réaction selon l'invention peut être effectuée en milieu concentré (sans solvant). Il est cependant préférable d'opérer au sein d'un solvant choisi parmi les composés inertes vis-à-vis des deux réactifs dans les conditions réactionnelles utilisées et ayant un point d'ébullition différent de celui du composé $R_FBr$ désiré. Comme solvants utilisables on peut mentionner, à titre non limitatif, l'eau, les alcools (par exemple le méthanol), les hydrocarbures aliphatiques halogénés comme le chlorure de méthylène, le chloroforme et le bromure de méthylène, ou d'autres solvants comme l'acétone, l'acétonitrile, le toluène.

La réaction selon l'invention peut être conduite à une température allant de la température ambiante jusqu'à environ 150°C. La température choisie doit cependant être inférieure à la température de décomposition du composé (I) mis en oeuvre. D'autre part, pour minimiser la formation concomitante de chlorure de perfluoroalkyle $R_F$-Cl, il est préférable d'opérer en-dessous de 60°C. A cet égard, il est particulièrement avantageux d'utiliser comme solvant le chlorure de méthylène dont le point d'ébullition (40°C) permet de réguler facilement la température du mélange réactionnel ; le chlorure de méthylène est en outre un excellent solvant du TBAB.

Un mode particulièrement préféré de réalisation du procédé selon l'invention consiste à effectuer l'introduction du composé (I) à basse température (20 à 30°C environ), à maintenir ensuite le mélange réactionnel à cette température jusqu'à un taux de transformation du sulfochlorure d'environ 80 % et enfin à terminer la réaction en chauffant au reflux.

On opère de préférence à la pression atmosphérique, mais on ne sortirait pas du cadre de la présente invention en travaillant sous pression sub- ou superatmosphérique.

Le procédé selon l'invention peut être mis en oeuvre aussi bien à partir d'un sulfochlorure $R_F$-$SO_2Cl$ brut que d'un sulfochlorure purifié par distillation. Sans que cela nuise au rendement, on peut opérer en introduisant le sulfochlorure dans le composé (I) ou, inversement, en introduisant le composé (I) dans le sulfochlorure.

L'isolement du bromure de perfluoroalkyle $R_F$-Br peut être effectué selon les techniques habituelles, par exemple par décantation du mélange réactionnel, lavage alcalin et distillation fractionnée.

Au cas où il subsiste un peu de sulfochlorure résiduel dans le produit décanté, on peut avantageusement, avant l'étape de lavage alcalin, ajouter au produit décanté (de préférence encore chaud) une faible quantité de composé I frais (environ 1 à 10 fois la stoéchiométrie du sulfochlorure résiduel présent) et procéder à une nouvelle décantation.

Les exemples suivants illustrent l'invention sans la limiter.

## EXEMPLE 1

Dans un réacteur en verre de 500 ml équipé d'une agitation, d'une ampoule de coulée, d'un réfrigérant et d'un dispositif de chauffage, on introduit 89 g de chlorure de méthylène et 89 g (0,275 mole) de TBAB, ce qui conduit à une solution homogène liquide à température ambiante.

Cette solution est chauffée à 50°C, puis on introduit en une heure une solution de 130 g de $C_8F_{17}SO_2Cl$ de pureté $\geqq$ 99 % (soit 0,250 mole) dans 30 g de chlorure de méthylène.

Après la fin de l'addition, on maintient la température au reflux (50-55°C) pendant 3 heures.

Après une purge par de l'azote, on laisse décanter le mélange réactionnel et on récupère ainsi :

- une phase supérieure (216 g) constituée de chlorure de méthylène, de $SO_2$ (0,231 mole) et de chlorure (0,249 mole) ;
- une phase inférieure pratiquement incolore (122 g) dont l'analyse CPV indique la composition suivante :

$C_8F_{17}Br$ = 85,1 % (soit un rendement de 83 %)

$CH_2Cl_2$ = 9,4 %
$C_8F_{17}SO_2Cl$ = 0,8 %
$C_8F_{17}Cl$ = 3,8 %

Cette phase inférieure est ensuite lavée avec 12 g d'une solution aqueuse à 10 % en poids d'hydroxyde de sodium. Après décantation et lavage à l'eau, on procède à une distillation fractionnée à pression atmosphérique. On obtient ainsi le bromure de perfluorooctyle avec une pureté supérieure à 99 %.

**EXEMPLE 2**

On opère dans le même appareillage qu'à l'exemple 1. Le réacteur est chargé avec 130 g de $C_8F_{17}SO_2Cl$ et 80,5 g de TBAB. Le mélange réactionnel est chauffé lentement (une heure) jusqu'à 125°C, puis est maintenu pendant 4 heures à cette température.

Par décantation à chaud on recueille 2 phases :

- une phase supérieure (90 g) contenant 0,217 mole d'ions $Cl^{\ominus}$ ;
- une phase inférieure jaune clair (115,5 g) ayant la composition suivante (analyse CPV) :

$C_8F_{17}Br$ = 89,3 % (soit un rendement de 82,5 %)
$C_8F_{17}SO_2Cl$ $\leqq$ 0,1 %
$C_8F_{17}Cl$ = 9,1 %

**EXEMPLE 3**

On opère dans le même appareillage qu'à l'exemple 1. Le réacteur est chargé avec une solution de 80,5 g (0,25 mole) de TBAB dans 87 g de chlorure de méthylène.

On introduit ensuite très rapidement une solution de 130 g de $C_8F_{17}SO_2Cl$ de pureté $\geqq$ 99 % (soit 0,25 mole) dans 46 g de chlorure de méthylène, puis on maintient sous agitation et à une température entre 20 et 30°C pendant 24 heures.

Après décantation du mélange réactionnel, on recueille :

- une phase supérieure (216 g) constituée majoritairement de chlorure de méthylène et contenant 0,232 mole de $SO_2$ et 0,231 mole de chlorure
- une phase inférieure pratiquement incolore (127 g) ayant la composition suivante :

$C_8F_{17}Br$ = 84,3 % (soit un rendement de 85,5 %)
$CH_2Cl_2$ = 9,1 %
$C_8F_{17}SO_2Cl$ = 4,1 %
$C_8F_{17}Cl$ = 2,3 %

**EXEMPLE 4**

Dans un réacteur de 100 ml équipé de la même manière qu'à l'exemple 1, on charge 46,5 g de chlorure de méthylène et 18,65 g (55 millimoles) de bromure de tétrabutylphosphonium. On chauffe la solution à 40°C (reflux de $CH_2Cl_2$) et on introduit en une heure une solution de 26 g (50 millimoles) de $C_8F_{17}SO_2Cl$ dans 6,5 g de chlorure de méthylène.

On maintient ensuite au reflux (environ 42°C) pendant 6 heures. Après décantation on recueille :

- une phase supérieure (75 g) constituée majoritairement de chlorure de méthylène et contenant 45 millimoles de $SO_2$ et 45 millimoles de chlorure
- une phase inférieure pratiquement incolore (22 g) ayant la composition suivante :

$C_8F_{17}Br$ = 78,6 % (soit un rendement de 69 %)
$CH_2Cl_2$ = 14,4 %
$C_8F_{17}SO_2Cl$ = 4,1 %
$C_8F_{17}Cl$ = 1,5 %

**EXEMPLE 5**

Dans un réacteur de 1 litre équipé comme à l'exemple 1, on charge 652 g d'une solution de $C_8F_{17}SO_2Cl$ à 20 % de $CH_2Cl_2$ (soit 1 mole de sulfochlorure), puis on introduit en une heure à une température comprise entre 20 et 30°C une solution de 355 g de TBAB (1,1 mole) dans 355 g de chlorure de méthylène.

On maintient sous agitation pendant environ 12 heures à une température comprise entre 20 et 30°C, puis on porte au reflux (50 ± 2°C) pendant 12 heures.

Par décantation, on sépare deux phases organiques. La phase organique inférieure (477 g), constituée

essentiellement de bromure de perfluorooctyle avec un peu (3,9 %) de sulfochlorure résiduel, est reprise par 50 g d'une solution à 50 % de TBAB dans $CH_2Cl_2$, puis chauffée à reflux (60°C environ) pendant 2 heures.

Après décantation et lavage à l'eau, on récupère une phase organique (468 g) contenant 94,8 % de bromure de perfluorooctyle (rendement : 89 %) et moins de 0,1 % de sulfochlorure. Ce produit peut être purifié par distillation comme à l'exemple 1.

## EXEMPLE 6 A 8

En suivant le mode opératoire de l'exemple 1, on a fait réagir le sulfochlorure $C_8F_{17}SO_2Cl$ avec d'autres composés bromés de formule (I), à savoir :

- exemple 6 : bromure de benzyl triméthyl ammonium
- exemple 7 : bromhydrate de pyridine
- exemple 8 : bromhydrate de tripropylamine

Les conditions opératoires et les résultats obtenus sont rassemblés dans le tableau suivant.

| EXEMPLE | 6 | 7 | 8 |
|---|---|---|---|
| Quantités utilisées pour : | | | |
| - composé (I) | 0,22 mole | 0,31 mole | 0,32 mole |
| - $C_8F_{17}SO_2Cl$ | 0,2 mole | 0,28 mole | 0,31 mole |
| - $CH_2Cl_2$ (a) | 190 g | 180 g | 140 g |
| Durée de réaction (b) | 6,75 h | 6,25 h | 7,75 h |
| Température | 42°C | 42°C | 47°C |
| Résultats: | | | |
| - Produit récupéré (c) | 108,6 g | 151,6 g | 150 g |
| - Taux de transformation du sulfochlorure | 93 % | 71 % | 100 % |
| - Rendement en $C_8F_{17}Br$ (d) | 57 % | 70 % | 66 % |

(a) : 128 g par mole de sulfochlorure et le reste pour le composé (I)

(b) : dont 1 heure pour l'introduction de la solution de sulfochlorure

(c) : phase inférieure après décantation

(d) : rendement par rapport au sulfochlorure transformé

## EXEMPLE 9

Dans le même appareillage qu'à l'exemple 1, on charge une solution de 89 g de TBAB dans 89 g d'eau, Dans cette solution chauffée à 50°C, on introduit en une heure 130 g de sulfochlorure $C_8F_{17}SO_2Cl$. On maintient ensuite à 50°C pendant 6 heures, puis on porte le mélange au reflux (101°C) et l'y maintient pendant 6 heures.

Après décantation, on récupère une phase inférieure (122 g) contenant 48 % de bromure de perfluorooctyle.

**EXEMPLE 10**

Dans le même appareillage qu'à l'exemple 1, on charge une solution de 89 g de TBAB dans 89 g de chlorure de méthylène, puis on introduit en une heure, à une température comprise entre 25 et 30°C, 105 g du sulfochlorure $C_6F_{13}SO_2Cl$ (0,25 mole). On maintient sous agitation pendant 12 heures a température ambiante, puis pendant 12 heures au reflux (54°C).

Après décantation, on récupère 70 g d'un produit contenant 89,7 % de bromure de perfluorohexyle.

**EXEMPLE 11**

On opère comme à l'exemple 10, mais en remplaçant le sulfochlorure $C_6F_{13}SO_2Cl$ par 80 g du sulfochlorure $C_4F_9SO_2Cl$.

En fin de réaction, on récupère après décantation un produit ayant une pureté CPV (hors solvant $CH_2Cl_2$) de 96,5 %.

## Revendications

1. Procédé de préparation des bromures de perfluoroalkyle, caractérisé en ce qu'il consiste à faire réagir un chlorure de perfluoroalcane-sulfonyle avec une quantité au moins équimolaire d'un composé de formule générale :

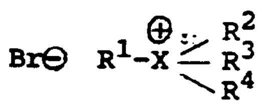

(I)

dans laquelle X représente un atome d'azote ou de phosphore, les symboles $R^1$, $R^2$, $R^3$ et $R^4$ sont identiques ou différents et représentent chacun un radical hydrocarboné éventuellement substitué, l'un de ces symboles $R^1$ à $R^4$ pouvant aussi être un atome d'hydrogène et/ou trois d'entre eux ensemble avec X pouvant former un noyau pyridine.

2. Procédé selon la revendication 1, dans lequel les radicaux hydrocarbonés sont des radicaux alkyle, aryle ou aralkyle éventuellement substitués.

3. Procédé selon la revendication 1, dans lequel le composé de formule (I) est le bromure de tétrabutylammonium.

4. Procédé selon l'une des revendications 1 à 3, dans lequel on opère au sein d'un solvant inerte ayant un point d'ébullition différent du composé $R_FBr$ désiré.

5. Procédé selon la revendication 4, dans lequel le solvant est le chlorure de méthylène.

6. Procédé selon l'une des revendications 1 à 5, dans lequel on utilise un excès de composé de formule (I), cet excès pouvant aller jusqu'à environ 10 %.

7. Procédé selon l'une des revendications 1 à 6, dans lequel on opère à une température allant de la température ambiante jusqu'à environ 150°C, de préférence à une température inférieure à 60°C.

8. Procédé selon la revendication 7, dans lequel on effectue l'introduction du composé (I) à une température d'environ 20 à 30°C, puis on maintient le mélange réactionnel à cette température jusqu'à la transformation d'environ 80 % du sulfochlorure, et on termine la réaction en chauffant au reflux.

9. Procédé selon l'une des revendications 1 à 8, dans lequel on part du chlorure de perfluorooctane-sulfonyle pour former le bromure de perfluorooctyle.

10. Procédé selon l'une des revendications 1 à 8, dans lequel on part du chlorure de perfluorohexane-sulfonyle pour former le bromure de perfluorohexyle.

## Patentansprüche

1. Verfahren zur Herstellung von Perfluoralkylbromiden, dadurch gekennzeichnet, daß dieses in der Reaktion eines Perfluoralkansulfonylchlorids mit einer mindestens äquimolaren Menge einer Verbindung der allgemeinen Formel:

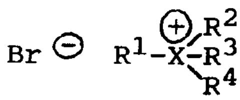

(I)

besteht, wobei X ein Stickstoff- oder Phosphoratom darstellt, die Symbole $R^1$, $R^2$, $R^3$ und $R^4$ identisch oder von einander verschieden sind, jedes ein gegebenenfalls substituiertes Kohlenwasserstoffradikal darstellen, und wobei eines dieser Symbole $R^1$ bis $R^4$ auch ein Wasserstoffatom und/oder drei von ihnen zusammen mit X einen Pyridinring bilden können.

2. Verfahren nach Anspruch 1, in dem die Kohlenwasserstoffradikale gegebenenfalls substituierte Alkyl-, Aryl- oder Aralkylradikale sind.

3. Verfahren nach Anspruch 1, in dem die Verbindung der Formel (I) Tetrabutylammoniumbromid ist.

4. Verfahren nach einem der Ansprüche 1 bis 3, in dem man in einem inerten Lösemittel arbeitet, dessen Siedepunkt von dem der erwünschten Verbindung $R_FBr$ verschieden ist.

5. Verfahren nach Anspruch 4, in dem das Lösemittel Methylenchlorid ist.

6. Verfahren nach einem der Ansprüche 1 bis 5, in dem man einen Überschuss der Verbindung der Formel (I) verwendet, wobei dieser Überschuss bis zu ungefähr 10 % betragen kann.

7. Verfahren nach einem der Ansprüche 1 bis 6, in dem man bei einer Temperatur arbeitet, die zwischen Raumtemperatur und 150°C liegen kann, vorzugsweise bei einer Temperatur unterhalb 60°C.

8. Verfahren nach Anspruch 7, in dem die Zugabe der Verbindung (I) bei einer Temperatur von ungefähr 20 bis 30°C durchgeführt, das Reaktionsgemisch dann bei dieser Temperatur bis zu einer Umsetzung des Sulfochlorids von ungefähr 80 % belassen wird, bevor man die Reaktion durch Erhitzen zum Rückfluss beendet.

9. Verfahren nach einem der Ansprüche 1 bis 8, in dem vom Perfluoroctansulfonylchlorid ausgegangen wird um Perfluoroctylbromid herzustellen.

10. Verfahren nach einem der Ansprüche 1 bis 8, in dem vom Perfluorhexansulfonylchlorid ausgegangen wird um Perfluorhexylbromid herzustellen.

## Claims

1. Process for the preparation of perfluoroalkyl bromides, characterised in that it consists in reacting a perfluoroalkanesulphonyl chloride with an at least equimolar amount of a compound of general formula:

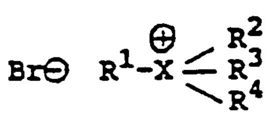

(I)

in which X represents a nitrogen or phosphorus atom and the symbols $R^1$, $R^2$, $R^3$ and $R^4$, which may be identical or different, each represent an optionally substituted hydrocarbon radical, it also being possible for one of these symbols $R^1$ to $R^4$ to be a hydrogen atom and/or it being possible for three of them together with X to form a pyridine ring.

**2.** Process according to Claim 1, in which the hydrocarbon radicals are optionally substituted alkyl, aryl or aralkyl radicals.

**3.** Process according to Claim 1, in which the compound of formula (I) is tetrabutylammonium bromide.

**4.** Process according to one of Claims 1 to 3, in which the reaction is carried out in an inert solvent which has a boiling point different from the desired compound $R_FBr$.

**5.** Process according to Claim 4, in which the solvent is methylene chloride.

**6.** Process according to one of Claims 1 to 5, in which an excess of compound of formula (I) is used, it being possible for this excess to be up to about 10 %.

**7.** Process according to one of Claims 1 to 6, in which the reaction is carried out at a temperature ranging from ambient temperature up to about 150°C, preferably to a temperature below 60°C.

**8.** Process according to Claim 7, in which the introduction of the compound (I) is carried out at a temperature of about 20 to 30°C, the reaction mixture is then kept at this temperature until about 80 % of the sulphonyl chloride has been converted and the reaction is completed by heating to reflux.

**9.** Process according to one of Claims 1 to 8, in which perfluorooctanesulphonyl chloride is used as starting material to form perfluorooctyl bromide.

**10.** Process according to one of claims 1 to 8, in which perfluorohexanesulphonyl chloride is used as starting material to form perfluorohexyl bromide.